**Europäisches Patentamt**

(19) **European Patent Office**

**Office européen des brevets**

(11) Numéro de publication: **0 107 222**
**A1**

(12) # DEMANDE DE BREVET EUROPEEN

(21) Numéro de dépôt: **83201355.1**

(22) Date de dépôt: **22.09.83**

(51) Int. Cl.³: **A 61 F 1/00**

(30) Priorité: **24.09.82 CH 5637/82**

(43) Date de publication de la demande: **02.05.84**
**Bulletin 84/18**

(84) Etats contractants désignés: **AT BE DE FR GB IT NL SE**

Demandeur: **Jeanjacquot, Alain, Châlet de la Pierre au Roy, F-74560 Monnetier-Mornex (FR)**
Demandeur: **Meyer, Pierre, 3, rue des Granges, CH-1204 Genève (CH)**
Demandeur: **Von Segesser, Ludwig, 31, rue Dancet, CH-1205 Genève (CH)**
Demandeur: **Hahn, Charles, Professeur, Centre de Chirurgie Cardiaque, CH-1261 Arzier (VD) (CH)**

(72) Inventeur: **Buchs, Jean-Bernard, Professeur, 17, Route de Florissant, CH-1206 Genève (CH)**
Inventeur: **Jeanjacquot, Alain, Châlet de la Pierre au Roy, F-74560 Monnetier-Mornex (FR)**
Inventeur: **Meyer, Pierre, 3, rue des Granges, CH-1204 Genève (CH)**
Inventeur: **Von Segesser, Ludwig, 31, rue Dancet, CH-1205 Genève (CH)**
Inventeur: **Hahn, Charles, Professeur, Centre de Chirurgie Cardiaque, CH-1261 Arzier (VD) (CH)**

(71) Demandeur: **Buchs, Jean-Bernard, Professeur, 17, Route de Florissant, CH-1206 Genève (CH)**

(74) Mandataire: **Silhol, Christian Maurice Alfred et al, c/o BUGNION S.A. Conseils en Propriété Industrielle 10, route de Florissant Case Postale 375, CH-1211 Genève 12 - Champel (CH)**

(54) Prothèse pour organe artificiel hybride.

(57) La prothèse pour organe artificiel hybride totalement transplantable est composée de deux parties (A, B), la première (A) comprenant un tube (10) destiné à être raccordé à une artère du receveur, des micro-tubes (11) en PAN montés sur un manchon (12) en silicone et reliés par une de leurs extrémités à l'intérieur (14) d'une cupule (13) reliée elle-même à un cathéter (15), la seconde partie (B) étant constituée par une enveloppe (16) en polytétrafluoroéthylène se prolongeant par un tube souple (17) destiné à être raccordé à une veine du receveur. Immédiatement avant l'implantation, on remplit les micro-tubes (11) de matériel vivant prélevé sur un organe sain, on ferme ces micro-tubes (11) avec une céramique polymérisant à froid, puis on assemble lesdites parties (A, B) en engageant l'extrémité de l'enveloppe (16) sur un manchon (12) et en la collant au moyen d'une couche fraiche de silicone. On raccord ensuite, au cours de l'implantation, les tubes (10, 17) au système vasculaire. A partir de ce moment, les micro-tubes (11) flottent dans le flux sanguin parcourant l'enveloppe (16) et les parois de ces tubes (11) sont traversées exclusivement par du plasma qui contient les substances nécessaires à la survie des cellules composant le matériel vivant contenu dans les micro-tubes (11). Les sécrétions de ces cellules sont recueillies dans la cupule (13), puis acheminées par le cathéter (15) à l'endroit du corps où elles sont nécessaires.

- 1 -

## Prothèse pour organe artificiel hybride.

Il existe deux grands concepts pour remplacer la déficience fonctionnelle d'un organe humain. Le premier est la tranplantation d'un organe vivant provenant d'un donneur, le second est l'implantation d'un organe totalement artificiel.

Ces deux concepts présentent des inconvénients, notamment des risques de rejet pour le premier et le manque de fiabilité technologique pour le second.

Les organes artificiels hybrides (OAH) relèvent d'un troisième concept qui vise à combiner les avantages des deux concepts fondamentaux sans en avoir les inconvénients.

Les organes artificiels hydrides se composent d'une prothèse et de matière vivante qui doit pouvoir survivre à l'intérieur de l'organisme du receveur, s'y répliquer ou du moins pouvoir suivre normalement le processus de réparation nécessaire à un vieillissement physiologique. Cela signifie que la matière vivante doit pouvoir bénéficier de l'apport de substances indispensables contenues dans le sang, mais en même temps être protégée contre les agressions des agents de la réponse immunologique.

La réalisation d'un organe artificiel hybride pose donc le problème de la mise en oeuvre, au niveau de la matière vivante, d'un processus de filtration du sang qui permette aux substances nutritives contenues dans le plasma de pénétrer seules et en quantité suffisante dans cette matière.

Ce processus, qui doit naturellement s'effectuer en continu, est obtenu au moyen d'une paroi présentant des propriétés de porosité telle qu'elle est perméable au plasma sanguin, tandis qu'elle constitue une barrière ne laissant pas passer les macro-molécules et les cellules immuno-compétentes.

La présente invention a pour objet une prothèse d'organe artificiel hybride totalement transplantable qui a été conçue en vue de remplir les conditions suivantes :

- garantir la survie de la matière vivante,
- être bien tolérée,
- être implantable périphériquement,
- présenter un minimum d'inertie fonctionnelle,
- pouvoir être remplacée facilement,
- être utilisable pour des organes divers.

Cette prothèse est caractérisée par le fait qu'elle est constituée de deux parties présentant des moyens permettant de les raccorder respectivement à une artère et à une veine du receveur, la première partie étant constituée par des micro-tubes à parois poreuses, reliés par l'une de leurs extrémités à un collecteur relié lui-même à un cathéter, la seconde partie étant constituée par une enveloppe, le tout de manière qu'après avoir, immédiatement avant l'implantation, rempli les micro-tubes de matériel vivant prélevé sur un organe sain, fermé ces micro-tubes à leurs secondes extrémités, puis assemblé lesdites parties, enfin raccordé l'organe artificiel hybride ainsi formé à une artère et à une veine, lesdits micro-tubes soient placés au moins sur une partie de leurs longueurs dans un espace étanche dans lequel circule un flux sanguin, de façon que les parois poreuses des micro-tubes soient traversées exclusivement par des substances, venues du sang, nécessaires à la survie du matériel vivant, et que les sécrétions produites par ce matériel dans les micro-tubes s'écoulent dans ledit collecteur.

Le dessin ci-annexé représente, à titre d'exemple, une forme d'exécution de l'objet de l'invention constituant la prothèse d'un pancréas artificiel hybride totalement transplantable.

La figure 1 est une vue schématique des deux parties de la prothèse telles qu'elles sont livrées par le fabricant.

La figure 2 est une vue analogue de la prothèse immédiatement avant son implantation.

La figure 3 est une section par la ligne III-III de la figure 3.

La figure 4 est une section par la ligne IV-IV d'un détail de la figure 3.

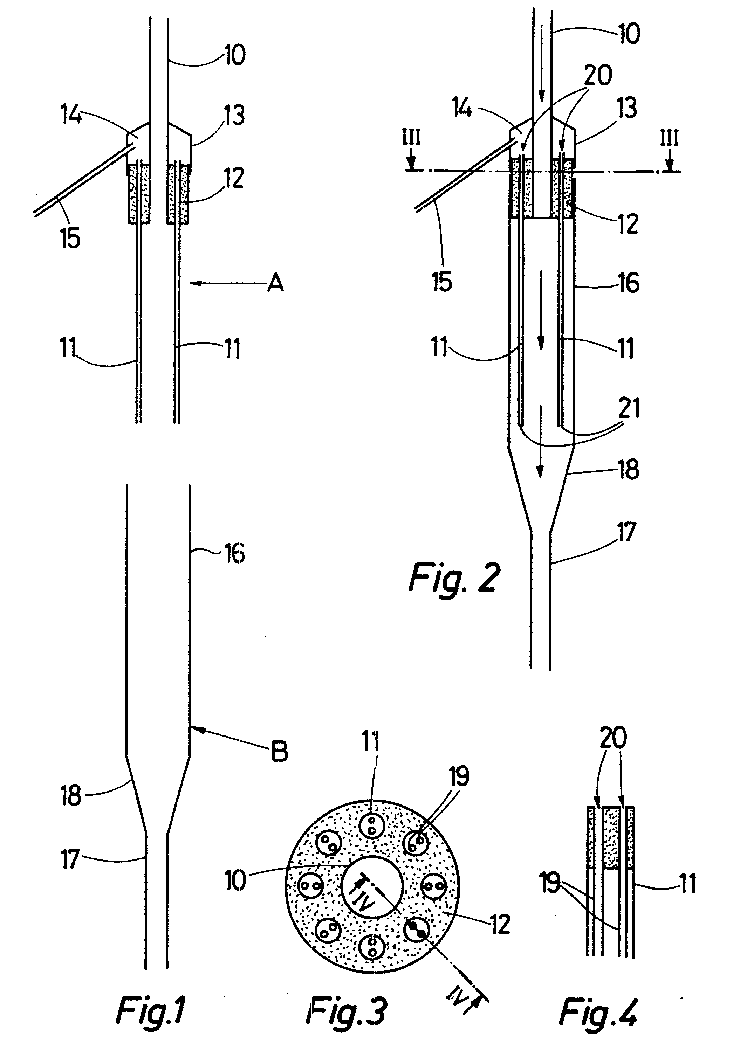

La prothèse représentée est composée initialement de deux parties distinctes A et B (figure 1).

La partie A est constituée par un tube souple 10 destiné à être raccordé à une artère du receveur, par une couronne de micro-tubes 11 à parois poreuses fixés sur l'extrémité du tube 10 par l'intermédiaire d'un manchon 12 en silicone, et par un collecteur 13 en forme de cupule, monté à cheval sur le tube 10 et sur l'extrémité du manchon 12. Les extrémités correspondantes des micro-tubes 11 débouchent directement dans l'espace intérieur 14 de la cupule du collecteur 13 relié à un cathéter 15. La partie B est constituée par une enveloppe cylindrique 16, plus longue que les micro-tubes 11, se prolongeant par un tube souple 17, de diamètre réduit, destiné à être raccordé à une veine du receveur. L'enveloppe 16 et le tube 17 sont reliés par un tronçon tronconique 18. En variante, ce tronçon 18 pourrait présenter une forme différente correspondant à une fonction mathématique quelconque. Les deux parties A et B sont destinées à être assemblées, immédiatement avant l'implantation, après que les micro-tubes 11 aient été remplis d'une certaine quantité d'ilots de Langerhans prélevés sur un pancréas animal sain puis fermés par tous moyens appropriés, mais avantageusement à l'aide d'un matériau céramique polymérisable.

L'assemblage est réalisé, comme illustré à la figure 2, en engageant l'extrémité ouverte de l'enveloppe 16 sur le manchon 12 recouvert d'une couche fraiche de silicone.

L'implantation de l'organe artificiel hybride s'effectuera avantageusement dans une zone périphérique et le cathéter 15 sera relié par voie sous-cutanée au péritoine du receveur.

A partir du moment où les tubes 10 et 17 sont reliés au système vasculaire, l'enveloppe 16 est parcourue, dans le sens des flèches, par un flux sanguin dans lequel flottent les micro-tubes 11. Ce flux s'écoule selon un régime laminaire et se répartit uniformément sur toute la surface des micro-tubes 11. La structure et la porosité des parois des micro-tubes 11 sont choisies de manière que ces parois ne puissent être traversées que par le plasma du sang qui contient les substances nécessaires à la vie des cellules composant les ilôts de Langerhans responsables, entre autre, de la sécrétion de l'insuline.

Dans ces conditions, les cellules de cette espèce, contenues dans les micro-tubes 11, reçoivent les substances nécessaires à leur vie et elles secrètent naturellement dans ces micro-tubes 11 de l'insuline qui est collectée dans la cupule du collecteur 13 avant d'être acheminée, par le cathéter 15 dans le péritoine.

Pour éviter que des cellules appartenant aux ilôts de Langerhans puissent, entraînées par l'insuline, s'échapper dans la cupule du collecteur 13, les extrémités correspondantes des micro-tubes 11 sont calibrées au moyen de tubes microscopiques 19 de diamètres tels qu'ils laissent passer l'insuline et retiennent les cellules composant les ilôts de Langerhans (figure 4). Dans l'état actuel de la technologie, les matériaux qui sont les mieux appropriés pour la prothèse décrite sont les suivants :

- pour l'enveloppe 16, un matériau tel que le polytétrafluoréthylène connu sous la marque "GORE-TEX", ayant la particularité de favoriser une fibrinolyse ;
- pour les micro-tubes 11, un matériau tel que la polyacrylonitrile présentant la propriété de n'induire que peu de réactions d'intolérance ;
- pour boucher les extrémités des micro-tubes 11, on emploie un matériau, tel qu'une céramique autopolymérisant à froid sans libération de substances toxiques au cours de cette transformation ;
- pour le collecteur 13 en forme de cupule, un matériau semi-rigide ;
- pour le manchon 12 et les autres joints, on utilise un matériau tel qu'une résine de silicones présentant la propriété d'être bio-compatible, par exemple du silicium connu sous la marque "SILASTIC".

L'invention n'est évidemment pas limitée à la forme d'exécution représentée au dessin et à l'application envisagée.

En particulier, elle pourrait être adaptée, sans que les principes sur lesquels elle repose soient modifiés, pour réaliser des organes artificiels hybrides remplaçant les fonctions thyroidiennes, parathyroidiennes, gonadiques, hypophysaires, surrénales, hépatiques et spléniques.

Revendications

1. Prothèse pour organe artificiel hybride totalement transplantable, caractérisée par le fait qu'elle est composée de deux parties (A, B) munies de moyens de raccordement, respectivement, à une artère et à une veine du receveur, la première partie (A) étant constituée d'une pluralité de micro-tubes (11) à parois poreuses, reliés par l'une de leur extrémités (20) à un collecteur (13) relié lui-même à un catheter (15), la seconde partie (B) étant constituée par une enveloppe (16), et par le fait que juste avant de procéder à l'implantation de la prothèse, on remplit les micro-tubes (11) de matériel vivant prélevé sur un organe sain, on ferme les secondes extrémités (21) de ces micro-tubes, on assemble hermétiquement les deux parties (A) et (B), on raccorde l'organe artificiel hybride ainsi formé à une artère et à une veine de façon que les micro-tubes soient placés, au moins sur une partie de leur longueur, dans un espace étanche où circule un flux sanguin, leurs parois étant traversées exclusivement par des substances, venues du sang, nécessaires à la survie du matériel vivant, les sécrétions produites par ce matériel dans les micro-tubes s'écoulant dans le collecteur relié au catheter.

2. Prothèse selon la revendication 1, caractérisée par le fait que la partie (B) enveloppant les micro-tubes (11) est cylindro-conique, par le fait que les moyens de raccordement de la partie (A) à une artère sont constitués par un tube cylindrique (10), et par le fait que les moyens de raccordement de la partie (B) à une

veine sont constitués par un tube souple (17) constituant l'enveloppe (16).

3. Prothèse selon la revendication 2, caractérisée par le fait que les micro-tubes (11) sont fixés en couronne sur un manchon (12) monté sur l'extrémité du tube (10) débouchant dans l'enveloppe (16), par le fait que le collecteur (13) est constitué par une cupule montée sur ledit tube (10), par le fait que les micro-tubes (11) traversent le manchon (12) de manière que leurs extrémités ouvertes (20) débouchent dans l'espace intérieur (14) de la cupule du collecteur (13), et par le fait que le manchon (12) s'étend au-delà du collecteur (13) de manière à servir de joint étanche entre l'enveloppe (16) et ledit tube (10) au moment de l'assemblage des deux parties (A, B) de la prothèse.

4. Prothèse selon la revendication 1, caractérisée par le fait que les extrémités ouvertes (20) des micro-tubes (11) sont calibrées de manière à ne pas laisser s'échapper le matériel vivant.

5. Prothèse selon la revendication 1, caractérisée par le fait que l'enveloppe (16) recouvrant les micro-tubes (11) est constituée par un matériau tel que le polytétrafluoroéthylène ayant la particularité de favoriser une fibrinolyse.

6. Prothèse selon la revendication 1, caractérisée par le fait que les micro-tubes (11) sont réalisés avec un matériau tel que le polyacrylonitrile présentant la propriété de n'induire que peu de réactions d'intolérance.

7. Prothèse selon la revendication 1, caractérisée par le fait que le manchon (12) est constitué par un matériau tel qu'une résine de silicones présentant la propriété d'être biocompatible.

8. Prothèse selon la revendication 1, caractérisée par le fait que le collecteur (13) en forme de cupule est réalisé en matériau semi-rigide.

9. Prothèse selon la revendication 1, caractérisée par le fait que les micro-tubes (11) sont obturés, après mise en place du matériel vivant, à leur extrémité (21) opposée à celle qui débouche dans la cupule du collecteur (13) par un matériau autopolymérisant à froid sans libération de substances toxiques au cours de cette transformation.

0107222

**Fig. 1**

**Fig. 2**

**Fig. 3**

**Fig. 4**

**Office européen des brevets**

**RAPPORT DE RECHERCHE EUROPEENNE**

EP 83 20 1355

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | DE-A-2 811 372 (CONNAUGHT LABORATORIES LTD.) <br> * Revendications 1, 3; figure 2 * | 1 | A 61 F 1/00 |
| | --- | | |
| Y | GB-A-1 479 002 (SPIELBERG) <br> * Revendications 1, 5; figures 14-16 * | 1 | |
| | --- | | |
| A | US-A-3 093 831 (JORDAN) | | |
| | --- | | |
| A | US-A-3 827 565 (MATSUMURA) | | |
| | --- | | |
| A | US-A-4 242 460 (CHICK) | | **DOMAINES TECHNIQUES RECHERCHES (Int. Cl. 3)** |
| | --- | | |
| A | FR-A-2 270 895 (COMMUNITY BLOOD COUNCIL OF GREATER NEW YORK INC.) | | A 61 F 1/00 <br> A 61 M 1/00 |
| | ----- | | |

Le présent rapport de recherche a été établi pour toutes les revendications

| Lieu de la recherche <br> BERLIN | Date d'achèvement de la recherche <br> 13-12-1983 | Examinateur <br> KANAL P K |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82